# FASCICULE DE BREVET EUROPEEN

(11) **EP 2 719 422 B1**
(45) Date de publication et mention de la délivrance du brevet: **18.02.2015**
(21) Numéro de dépôt: 13169168.5
(22) Date de dépôt: 24.05.2013
(51) Int. Cl.: A61N 1/05

(54) **Microsonde multipolaire de détection/stimulation implantable**
Implantierbare multipolare Mikrosonde zur Erfassung/Stimulation
Implantable multipolar detection/stimulation microprobe

(30) Priorité: 12.10.2012 FR 1259758
(43) Date de publication de la demande: 16.04.2014
(73) Titulaire: Sorin CRM SAS, 92140 Clamart Cedex (FR)
(72) Inventeur: Régnier, Willy, 91160 Longjumeau (FR); Shan, Nicholas, 91260 Juvisy sur Orge (FR); Ollivier, Jean-François, 91190 Villiers Le Bacle (FR); D'Hiver, Philippe, 92320 Chatillon (FR)
(74) Mandataire: Dupuis-Latour, Dominique

(56) Documents cités:
- EP-A1- 2 384 784
- EP-A1- 2 455 131

## Description

L'invention concerne de façon générale les "dispositifs médicaux implantables actifs" tels que définis par la Directive 90/385/CEE du 20 juin 1990 du Conseil des communautés européennes.

Cette définition inclut en particulier les implants cardiaques chargés de surveiller l'activité cardiaque et de générer des impulsions de stimulation, de défibrillation et/ou de resynchronisation en cas de trouble du rythme détecté par l'appareil, et/ou de détecter une activité électrique. Elle inclut aussi les appareils neurologiques, les implants cochléaires, les pompes de diffusion de substances médicales, les capteurs biologiques implantés, etc.

Ces dispositifs comportent un boitier généralement désigné "générateur", raccordé électriquement et mécaniquement à une ou plusieurs "sondes" intracorporelles munie(s) d'électrodes destinées à venir en contact avec les tissus sur lesquels on souhaite appliquer des impulsions de stimulation et/ou recueillir un signal électrique : myocarde, nerf, muscle...

La présente invention concerne plus précisément une microsonde de détection/stimulation destinée à être implantée dans des réseaux veineux, artériels ou lymphatiques.

Le principe actuel de stimulation électrique des tissus est basé sur un dispositif, appelé généralement "sonde", qui est un objet implanté au travers de divers vaisseaux, veineux, artériels ou lymphatiques notamment, et dont la fonction est de transmettre un signal électrique au tissu-cible tout en garantissant les propriétés suivantes :
- facilité d'implantation par le médecin dans un réseau de vaisseaux du patient, et en particulier facilité : à faire progresser la sonde dans les vaisseaux par poussée, à faire suivre à la sonde des trajets tortueux et passer des embranchements, et à transmettre des couples ;
- visibilité aux rayons X afin de permettre au médecin une navigation aisée à travers les vaisseaux du réseau sous radioscopie X ;
- atraumaticité de la sonde au niveau des veines, ce qui nécessite une grande souplesse de structure et l'absence de transition rigide ou d'angle vif ;
- aptitude à transmettre un signal électrique aux tissus et à faire des mesures électriques monopolaires ou multipolaires de manière stable ;
- biocompatibilité avec les tissus vivants pour une implantation à long terme ;
- capacité à se connecter à un appareil implantable source de transmission du signal ;
- aptitude à la stérilisation (rayons gamma, température...) sans subir de dommages ;
- biostabilité, en particulier résistance à la corrosion dans le milieu vivant et résistance à la sollicitation mécanique en fatigue liée aux mouvements du patient et des organes ; et
- compatibilité avec l'imagerie IRM, particulièrement importante en neurologie.

L'architecture actuelle des sondes répondant à ces besoins peut se résumer à une structure généralement creuse pour permettre le passage d'un mandrin ou d'un fil-guide, et comprenant des composants de type câbles conducteurs isolés, reliés à des électrodes mécaniques pour assurer la conductivité électrique, la radio-opacité, etc.

Il s'agit donc de sondes nécessitant un assemblage complexe d'un nombre important de pièces, de fils et d'isolants associés, créant des risques non négligeables de rupture compte tenu des contraintes mécaniques à long terme auxquelles elles sont exposées.

Des exemples de telles sondes sont donnés dans les US 6 192 280 A et US 7 047 082 A.

Parmi les difficultés rencontrées, on peut citer la gestion des gradients de raideur liés aux pièces mécaniques utilisées, qui affectent fortement les propriétés d'implantabilité et de résistance mécanique sur le long terme (fatigue). De plus, en termes de fatigue des assemblages, toute zone de transition de rigidité est susceptible d'induire des risques de fatigue, des difficultés à stériliser du fait de la présence de zones d'accès difficile, et des problèmes de tenue des jonctions de conducteurs au niveau de la liaison avec les électrodes et le connecteur.

Par ailleurs, la tendance clinique dans le domaine des sondes implantables est d'en réduire la taille afin de les rendre moins invasives et plus faciles à manipuler à travers les vaisseaux. La taille actuelle des sondes implantables est typiquement de l'ordre de 4 à 6 French (1,33 à 2 mm).

Cependant, il est clair que la réduction de la taille des sondes augmenterait leur complexité et imposerait des contraintes techniques génératrices de risques.

Pourtant, une telle réduction, à moins de 1,5 French (0,5 mm) par exemple, ouvrirait des perspectives d'applications médicales dans des domaines variés allant de la cardiologie à la neurologie en présence d'un réseau veineux, artériel ou même lymphatique, comme le réseau veineux cérébral ou le réseau veineux du sinus coronaire.

Aujourd'hui, la technologie de stimulation électrique a permis des avancées importantes dans le domaine de la neuromodulation, qui consiste à stimuler des zones-cibles du cerveau pour le traitement de la maladie de Parkinson, l'épilepsie et autres maladies neurologiques.

On pourrait donc imaginer parvenir avec ce type de technologie à traiter de nouvelles régions difficilement atteignables aujourd'hui, ceci au moyen de sondes de stimulation de petite taille, ou "microsondes", présentant une grande robustesse afin de garantir la biostabilité à long terme. Avec des microsondes de taille réduite, il est possible d'envisager notamment le passage d'anastomoses pour établir par exemple un dispositif de stimulation du ventricule gauche via deux zones distinctes.

Une telle technique permettrait également une approche moins invasive de ces traitements et surtout une efficacité supérieure des traitements administrés.

Il serait en outre possible de connecter une ou plusieurs microsondes à travers le réseau de vaisseaux considéré jusqu'à leur localisation-cible. Leur mise en place pourrait être effectuée, du fait de leur taille réduite, par des dispositifs de guidage utilisés aujourd'hui en neuroradiologie interventionnelle pour la libération de ressorts (*coils*) lors du traitement des anévrismes intracrâniens. En particulier, des microsondes de 1,5 French seraient compatibles avec des cathéters de diamètre interne 1,6 French. D'autre part, il existe un besoin de pouvoir disposer de sondes multipolaires dans des applications liées à la fonction de "repositionnement électronique", laquelle offre de nombreuses possibilités de stimulation des tissus à travers le choix et la polarisation de certaines lignes de conduction parmi plusieurs incluses dans la microsonde. Cette technique permet de programmer des zones de stimulation en fonction de la thérapie, propriété très intéressante en particulier en neurologie.

Le EP 2 455 131 A1 (Sorin CRM SAS) envisage une sonde groupant une pluralité de microsondes monopolaires distinctes. Le corps de sonde qui regroupe ces microsondes est doté sur sa longueur de plusieurs ouvertures latérales d'où émergent successivement des microcâbles respectifs. Chacun de ces microcâbles forme une ligne monopolaire s'étendant dans un vaisseau homologue du réseau coronarien, ce qui permet de stimuler au moyen d'une même sonde plusieurs vaisseaux (chacun par un microcâble monopolaire) et couvrir ainsi une large surface du ventricule gauche. Mais si l'on considère isolément chacune des microsondes, celles-ci ne comportent qu'un seul conducteur électrique et ne peuvent donc pas faire l'objet d'un repositionnement électronique.

Le but de la présente invention est de proposer une microsonde de petite taille qui serait en conformité avec les propriétés générales des sondes implantables telles qu'elles ont été énumérées plus haut, tout en en réduisant la complexité et, de ce fait, le cout final, et en permettant par ailleurs de réaliser une connexion multipolaire sur un même microcâble afin de pouvoir polariser des lignes indépendamment les unes des autres et stimuler les tissus au moyen d'électrodes disposées en différentes positions le long de la microsonde.

La taille de cette microsonde devrait notamment permettre d'atteindre des veinules de très petites dimensions, inaccessibles aujourd'hui avec des dispositifs de taille supérieure. La microsonde de l'invention devrait également faciliter sensiblement la navigabilité dans les réseaux veineux, artériels ou lymphatiques du fait de sa souplesse, amplifiée par ses petites dimensions.

De même, la microsonde de l'invention doit pouvoir répondre à un certains nombre d'exigences.

Au niveau de la fiabilité de la liaison mécanique et électrique conducteur/électrode, il s'agit d'éviter les conceptions risquant d'altérer la tenue mécanique immédiate du conducteur par des assemblages de composants de continuité électrique insérés entre différentes portions de conducteurs. Il est préférable en ce sens de privilégier la continuité physique du conducteur. Il convient également d'assurer une étanchéité maximum afin de ne pas exposer les conducteurs aux fluides corporels.

Au niveau des performances, il s'agit de :
- donner à la microsonde un diamètre extérieur compatible avec la taille des vaisseaux visés ;
- ne pas altérer localement la grande flexibilité de la microsonde afin de conserver une maniabilité maximale, notamment au passage à travers les petites veines ;
- assurer un profil isodiamétrique pour permettre le passage dans un cathéter implantable et les veines ; et
- faciliter et simplifier la procédure d'assemblage microsonde/cathéter. Conformément à l'invention, ce but est atteint grâce à une microsonde multipolaire de détection/stimulation destinée à être implantée dans des réseaux veineux, artériels ou lymphatiques.

Comme divulgué par le EP 2 455 131 A1 précité, la sonde comprend une pluralité de microcâbles, chacun comprenant un câble de coeur électriquement conducteur, apte à être relié à un pôle d'un générateur multipolaire d'un dispositif médical implantable actif, et une couche d'isolement en polymère entourant le câble de coeur et comprenant au moins une zone dénudée ménagée dans la couche d'isolement et destinée à former une électrode de détection/stimulation. Chaque câble de coeur est formé par une pluralité de brins élémentaires assemblés ensemble en un toron de brins respectif.

La sonde de l'invention est remarquable en ce que les torons de brins des différents microcâbles sont eux-mêmes assemblés ensemble en un toron de microcâbles, ce toron de microcâbles formant une partie active distale de stimulation multipolaire de la sonde, et en ce que la sonde est une microsonde, le diamètre de la partie active distale étant au plus égal à 1,5 French (0,5 mm).

Ainsi, on comprend qu'avec un diamètre ne dépassant pas 0,5 mm, la microsonde selon l'invention présente une grande flexibilité, favorable à sa manipulation par le médecin, notamment au cours de son implantation lorsqu'il s'agit par exemple de l'introduire dans des réseaux de vaisseaux avec de fortes tortuosités et de nombreux embranchements et d'éviter des traumatismes que pourraient engendrer des sondes beaucoup plus rigides, incompatibles avec les tissus.

Selon un mode de réalisation de l'invention, le câble de coeur est formé par une pluralité de brins élémentaires de 0,033 mm de diamètre. En particulier, un câble de coeur de 0,1 mm est formé par un toron de sept brins élémentaires de 0,033 mm de diamètre.

Le choix d'une structure multifilaire toronnée composée de brins élémentaires très fins pour réaliser les câbles de coeur permet d'augmenter leur résistance à la fatigue mécanique due aux mouvements du patient et des organes, sachant que la limite de rupture en flexion d'un fil est sensiblement inversement proportionnelle à son diamètre.

De manière à renforcer cette importante propriété de biostabilité, il y a avantage à ce que les brins élémentaires présentent une structure composite constituée d'un matériau structurant et d'un matériau radio-opaque. De préférence, le matériau structurant a une résistance à la fatigue intrinsèque élevée, comme par exemple un acier inox, un alliage de cobalt MP35N, un métal précieux, le titane ou un alliage NiTi connu notamment sous le nom de nitinol. À ces métaux chargés d'assurer les qualités mécaniques du câble de coeur s'ajoute un matériau radio-opaque destiné à rendre la microsonde visible aux rayons X lors de sa mise en place par le médecin. Le matériau radio-opaque peut être choisi parmi le tantale (Ta), le tungstène (W), l'iridium (Ir), le platine (Pt) et l'or (Au).

Pour réaliser le contact électrique avec les tissus et transmettre les signaux électriques de détection/stimulation, l'invention propose une solution qui consiste à utiliser les câbles de coeur pour former les électrodes de la microsonde en ménageant des zones dénudées dans une couche d'isolement entourant les câbles.

De préférence, la couche d'isolement est en polymère fluoré, éthylène tétrafluoroéthylène (ETFE) par exemple, et a une épaisseur de 25 µm.

Selon un mode de réalisation avantageux, la microsonde selon l'invention est constituée d'un toron de microcâbles, en particulier de sept microcâbles. L'invention prévoit également qu'une gaine en polymère thermorétractable, en polyester (PET) par exemple, entoure partiellement la microsonde.

Afin de limiter l'échauffement du câble de coeur par effet de peau lors d'une imagerie par IRM, l'invention recommande que les brins comprennent une couche extérieure en matériau à faible susceptibilité magnétique, inférieure à 2000.10⁻¹².m³.mole⁻¹. Le matériau à faible susceptibilité magnétique peut être, au choix, le tantale (Ta), le titane (Ti), le rhodium (Rh), le molybdène (Mo), le tungstène (W), le palladium (Pd) et l'or (Au). Selon une première variante de réalisation de la microsonde selon l'invention, une électrode de détection/stimulation comprend une bague conductrice fixée sur une zone dénudée de microcâble par réduction de son diamètre extérieur.

Afin d'éviter les contraintes sur la bague, les gaines et les torons, l'invention prévoit une seconde variante selon laquelle une électrode de détection/stimulation comprend, d'une part, une bague conductrice intérieure fixée sur une zone dénudée par réduction de son diamètre extérieur, et, d'autre part, une bague conductrice extérieure fixée sur la bague intérieure. Les bagues conductrices sont par exemple en platine iridié. Avantageusement, la bague extérieure est fixée sur la bague intérieure par soudure laser ou par collage au moyen d'une colle conductrice. L'invention prévoit également qu'une électrode de détection/stimulation comprend une bague conductrice fixée sur une zone dénudée par collage au moyen d'une colle conductrice.

Selon une autre variante, la zone dénudée s'étend sur 360°, sur une longueur correspondant à une spire de toron de microcâbles. Il est alors possible de réaliser sur la microsonde au moins un dipôle constitué de deux zones dénudées sur 360° disposées en regard sur deux microcâbles non consécutifs.

Enfin, il est avantageux que les extrémités distales des microcâbles soient décalées longitudinalement les unes par rapport aux autres, de manière à produire une diminution progressive du diamètre et introduire un gradient de raideur dans la partie la plus distale de la microsonde.

On va maintenant décrire des exemples de mise en oeuvre de l'invention, en référence aux dessins annexés où les mêmes références numériques désignent d'une figure à l'autre des éléments identiques ou fonctionnellement semblables.
La Figure 1 est une vue partielle en perspective d'une microsonde multipolaire de détection/stimulation conforme à l'invention.
La Figure 2 représente la microsonde de la Figure 1 avant mise en place de la gaine extérieure et des électrodes.
La Figure 3 est une section droite de la vue de la Figure 2.
La figure 4 est une vue en coupe d'un brin élémentaire d'un microcâble montré à la Figure 3.
La Figure 5 est une vue partielle en perspective d'une microsonde réalisée selon une première variante de réalisation de l'invention.
Les Figures 6a à 6c sont des vues illustrant les étapes de mise en place d'une bague intérieure sur un microcâble conformément à une deuxième variante de réalisation.
Les Figures 7a à 7c sont des vues illustrant les étapes de fixation par soudure laser d'une bague extérieure sur la bague intérieure des Figures 6a à 6b.
Les Figures 8a à 8c sont des vues illustrant les étapes de fixation par collage d'une bague extérieure sur une zone dénudée d'un microcâble.
La Figure 9 est une vue partielle en perspective montrant une électrode en partie distale du microcâble central de la microsonde de la Figure 1.
La Figure 10 est une vue partielle en perspective d'une microsonde réalisée selon une troisième variante de réalisation.
Les Figures 11a et 11b sont des vues montrant la réalisation d'un dipôle sur une microsonde conforme à l'invention.
Les Figures 12a et 12b illustrent un perfectionnement des modes de réalisation décrits aux Figures précédentes, permettant d'obtenir une réduction progressive du diamètre en extrémité distale de la sonde.

Les microsondes concernées par l'invention sont des microsondes multipolaires de détection/stimulation destinées à être implantées dans des réseaux veineux, artériels ou lymphatiques, et dont le diamètre ne dépasse pas 1,5 French (0,5 mm). Elles sont plus particulièrement adaptées aux applications mettant en oeuvre la fonction de repositionnement électronique, évoquée plus haut, qui implique une pluralité de lignes de conduction isolées et indépendantes, chacune reliée à un pôle du générateur d'un dispositif implantable via un connecteur de type IS-1 ou IS-4 pour une sonde cardiaque, ou même un plus grand nombre de pôles plus pour une sonde neurologique.

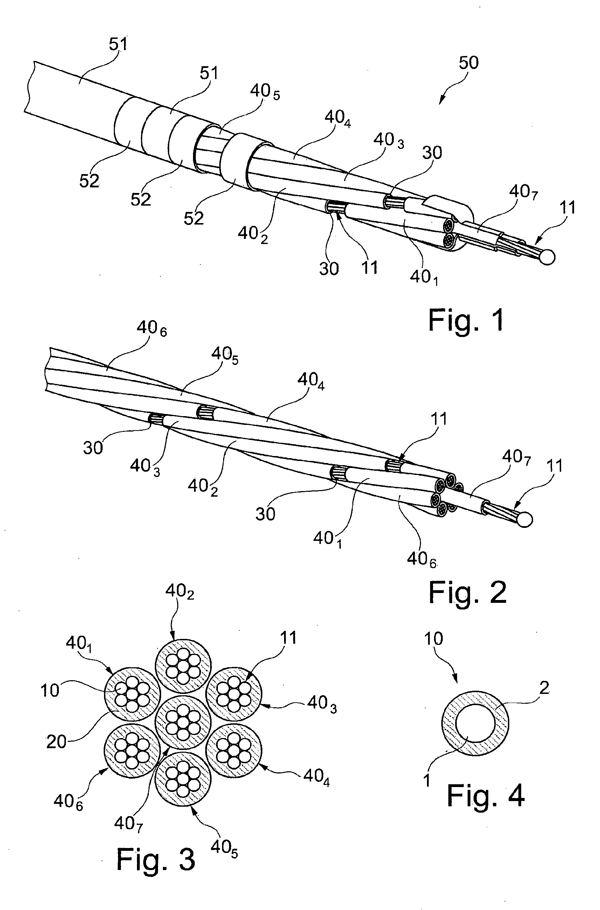

La microsonde 50 montrée sur la Figure 1 comprend une pluralité de sept microcâbles 40₁, 40₂, 40₃, 40₄, 40₅, 40₆, 40₇ assemblés selon un toron représenté plus spécialement à la Figure 2, chaque microcâble constituant pour la microsonde 50 une ligne de conduction reliée à un pôle du générateur.

Comme le montre la Figure 3, un microcâble comprend un câble 11 de coeur entouré d'une couche 20 d'isolement de manière à assurer l'isolation électrique des microcâbles entre eux.

Dans l'exemple de réalisation montré sur la section droite de la Figure 3, chaque câble 11 de coeur est formé par un toron de sept brins élémentaires 10 dont le diamètre est, par exemple, de 0,033 mm. Le diamètre d'un câble 11 de coeur est alors de 0,1 mm.

Il est proposé par l'invention d'utiliser des brins élémentaires 10 du type de celui représenté sur la Figure 4, c'est-à-dire comprenant une âme 1 en un matériau structurant comme un acier inox, un alliage de cobalt de la série MP35N, un métal précieux, le titane ou un alliage NiTi, de résistance à la fatigue élevée, le diamètre de 0,033 mm permettant en moyenne de garantir une résistance à la rupture en fatigue maximale dans les conditions extrêmes de contrainte auxquelles de telles structures peuvent être soumises.

Afin de garantir une visibilité aux rayons X suffisante pour l'implantation de la microsonde, il peut être nécessaire d'introduire le long du câble de coeur une quantité minimale de matériau radio-opaque 2 selon une structure composite conciliant résistance à la fatigue du câble et radio-opacité, la plupart des matériaux utilisés pour leur visibilité aux rayons X, à savoir le tantale (Ta), le tungstène (W), l'iridium (Ir), le platine (Pt) et l'or (Au) ne présentant pas en général une grande résistance à la fatigue.

On sait, d'une manière générale, que la compatibilité des dispositifs implantables avec les techniques médicales modernes d'imagerie, telles que l'IRM, est fondamentale pour garantir un traitement optimal du patient.

En effet, du fait de sa structure globalement métallique, la microsonde présente un risque d'échauffement lié aux courants induits par "effet de peau" à l'extérieur des brins élémentaires sous l'action du champ magnétique appliqué. Le faible diamètre donné aux brins est toutefois favorable à la dissipation thermique et réduit les effets d'échauffement dus à l'IRM. De plus, l'énergie thermique emmagasinée par les matériaux, déjà limitée en volume, peut encore être diminuée si les brins unitaires sont revêtus d'une couche extérieure en matériau à faible susceptibilité magnétique (la susceptibilité magnétique étant la faculté d'un matériau à s'aimanter sous l'action d'un champ magnétique extérieur).

Les matériaux les plus favorables dans cette application sont ceux dont la susceptibilité magnétique est inférieure à 2000.10⁻¹².m³.mole⁻¹, notamment le tantale (Ta), le titane (Ti), le rhodium (Rh), le molybdène (Mo), le tungstène (W), le palladium (Pd) et l'or (Au).

De préférence, l'épaisseur de la couche 20 d'isolement est de 0,025 mm (25 µm). On réalise ainsi des microcâbles de 0,150 mm de diamètre et un toron de sept microcâbles de diamètre égal à 0,45 mm.

Les caractéristiques requises pour la couche 20 d'isolement sont les suivantes :
- résistance à la fatigue,
- isolement électrique,
- biocompatibilité à long terme,
- biostabilité,
- possibilité de transformation et mise en oeuvre compatible avec le conducteur du câble de coeur.

Pour réaliser la couche 20 d'isolement, on privilégiera les matériaux à forte inertie chimique comme les polymères fluorés, qui présentent également une très bonne qualité d'isolation. Parmi ces composés, on peut citer en particulier l'ETFE (éthylène tétrafluoroéthylène).

Les procédés de réalisation de la couche 20 d'isolement sur le câble de coeur sont, par exemple, la co-extrusion sur le conducteur ou l'échauffement d'un tube thermorétractable.

Comme on peut le voir sur les Figures 1 et 2, les couches 20 d'isolement entourant les câbles 11 de coeur des microcâbles présentent au moins une zone dénudée 30 destinée à former, selon des procédés de réalisation qui seront décrits en détail plus loin, une électrode de détection/stimulation pour la microsonde 50, comme les électrodes 52 de la Figure 1. Les zones dénudées 30 sont obtenues notamment par la technique d'ablation laser.

Dans le cadre de stimulation dans une anastomose, par exemple, il est facilement envisageable d'ajouter une ou plusieurs séries d'électrodes sur chacun des microcâbles, ceci afin d'augmenter le nombre de points de stimulation :
- avec une électrode par microcâble périphérique, on obtient six électrodes périphériques et une à l'extrémité distale du microcâble central, soit sept pôles et sept électrodes ;
- avec deux électrodes par microcâble périphérique, on obtient douze électrodes périphériques et une à l'extrémité distale du microcâble central, soit sept pôles et treize électrodes ;
- avec trois électrodes par microcâble périphérique, on obtient dix-huit électrodes périphériques et une à l'extrémité distale du microcâble central, soit sept pôles et dix-neuf électrodes ;
- etc.

Cet exemple ne prend en compte qu'une seule électrode en extrémité distale du microcâble central 40₇. Il est cependant possible d'y placer une pluralité d'électrodes si le microcâble est allongé en partie distale. Dans ce cas, il n'y a pas de limitation au nombre d'électrodes, mais pour une microsonde constituée de sept microcâbles toronnés, le nombre de pôles est dépendant du nombre de microcâbles, soit ici sept pôles.

Une autre possibilité consiste à relier électriquement une pluralité de microcâbles à un même potentiel électrique, pour accroitre la fiabilité par cette redondance.

On peut ainsi prévoir par exemple, avec une structure 7x7 telle que celle illustrée Figure 3, de relier deux (ou trois) des sept microcâbles à un même pôle du générateur côté proximal, et côté distal à regrouper les électrodes de ces microcâbles dans une même zone de stimulation pour ne former qu'un point précis de stimulation - ou, inversement, de les éloigner entre elles de plusieurs centimètres pour créer une large surface de stimulation.

Enfin, la Figure 1 montre plus particulièrement que la microsonde 50 est entourée d'une gaine extérieure 51, sauf aux endroits occupés par les électrodes 52. Cette gaine 51 peut être en polymère thermorétractable, comme le polyéthylène téréphtalate (PET). Son épaisseur est typiquement de 0,025 mm (25 µm), ce qui correspond à un diamètre final de 0,5 mm soit 1,5 French pour la microsonde 50.

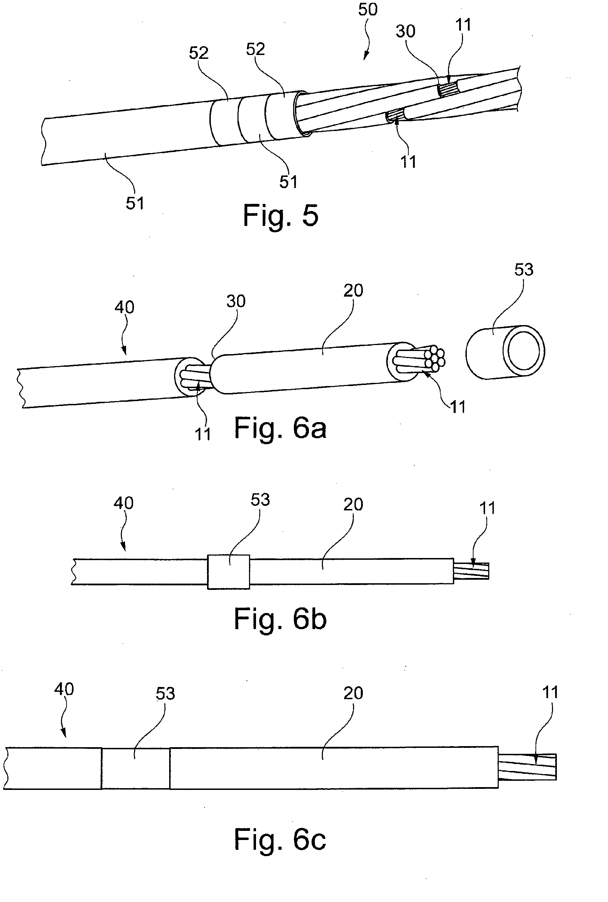

La Figure 5 illustre une première variante de réalisation de la microsonde 50 dans laquelle des bagues conductrices 52 de type platine/iridium 90/10 dont placées respectivement en regard d'une zone dénudée 30 (non visible sur la figure). Chaque bague 52 est fixée par réduction de son diamètre extérieur, par exemple par sertissage, jusqu'à la mise en contact local du diamètre intérieur de la bague 52 avec le câble 11 de coeur du microcâble dénudé.

La réduction du diamètre de la bague 52, obtenue par déformation de matière, entraine des contraintes intrinsèques à la bague mais aussi sur les couches 20 d'isolement et les câbles 11 de coeur. La matière des bagues 52 doit donc être suffisamment malléable afin de ne pas endommager les zones non dénudées.

L'assemblage de la microsonde 50 de la Figure 5 consiste à enfiler en alternance une longueur de gaine thermorétractable 51 puis une bague conductrice 52.

Il est à noter que les électrodes 52 sont de type annulaire et permettent une stimulation sur 360°.

L'ensemble des Figures 6a à 8c concerne des variantes de réalisation dont le but est de garantir l'intégrité des différentes couches d'isolement lors de la mise en place de l'électrode 52.

Dans la variante des Figures 6a à 7c, une première bague 53, dite bague intérieure, également en platine iridié, dont le diamètre intérieur est au moins égal au diamètre extérieur des microcâbles, soit 0,15 mm, pour un diamètre extérieur de 0,18 mm, est enfilée sur l'un des microcâbles 40 (Figure 6a) jusqu'à se trouver en regard d'une zone dénudée 30 (Figure 6b) et y être sertie (Figure 6c) de manière à réaliser le contact électrique avec le câble 11 de coeur et conserver l'isodiamètre. Le diamètre extérieur de la bague intérieure 53 est alors de 0,15 mm.

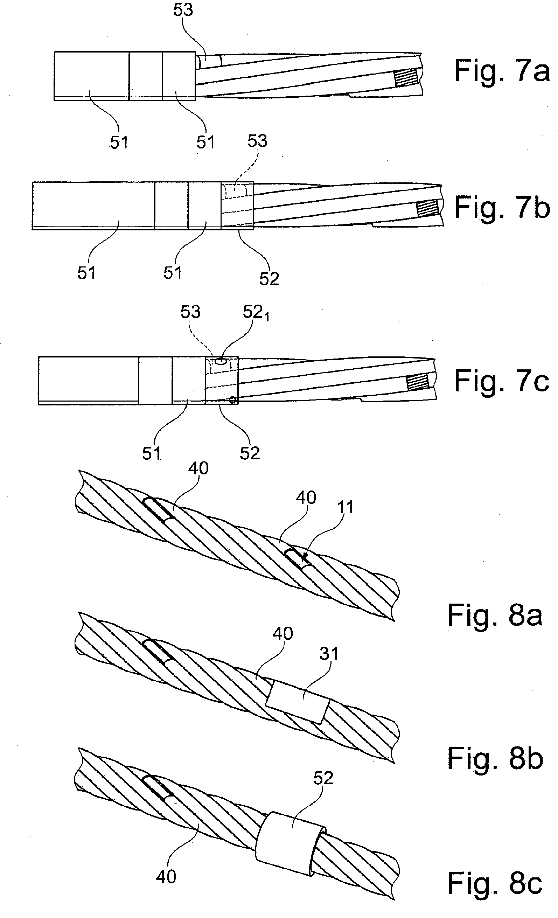

Cette bague 53 est ensuite utilisée comme repère visible (Figure 7a), sous binoculaire en position verticale, pour permettre la mise en place (Figure 7b) d'une deuxième bague 52, dite bague extérieure ou de stimulation, fixée à la bague intérieure 53.

Cette solution évite de soumettre les sept microcâbles à un effort de sertissage. Le contact électrique est alors reporté entre les deux bagues 52, 53. Les risques de dommage par sertissage sur les couches 20 d'isolement des microcâbles sont ainsi réduits.

Un trou 521 est ménagé dans la bague extérieure 52 (Figure 7c) afin de faciliter l'orientation et la mise en position des bagues 52, 53 pour un assemblage par soudure laser.

Selon la variante des Figures 8a à 8b, un microcâble 40 particulier présente une zone 30 dépourvue de couche 20 d'isolement (Figure 8a). Cette zone dénudée est recouverte par un dépôt 31 de colle conductrice (Figure 8b) permettant le montage et la mise en position d'une bague extérieure 52 qui devient l'électrode de stimulation en contact avec les tissus (Figure 8c).

La colle conductrice a pour fonction de transmettre un courant électrique entre les deux composants conducteurs et de préserver l'intégrité des couches d'isolement des microcâbles adjacents. Il n'y a pas de risque de faire fondre le polymère des couches d'isolement, ni de les couper ou de réduire localement leur épaisseur.

Une large gamme de colles biocompatibles et couramment utilisées dans les appareils médicaux implantables est disponible auprès de la société Epoxy Technology, Inc., par exemple sous la référence EPO-TEK (marque déposée).

Cette technique de collage peut s'appliquer également à l'assemblage d'une bague intérieure 53 et d'une bague extérieure 52, telles que décrites en regard des Figures 7a à 7c.

La structure située sous les bagues collées est constituée de brins métalliques rigides et non compressibles et de couches isolantes en polymère souple et donc compressible. À une échelle microscopique, il est possible de définir cette structure comme souple et pouvant varier en dimension et en géométrie. L'usage de colle permet de compenser ces déformations microscopiques grâce aux caractéristiques intrinsèques de la colle et d'introduire une relative souplesse dans l'assemblage des deux bagues et de rendre cette fixation moins cassante lorsqu'elle est soumise à des contraintes de traction, flexion ou torsion.

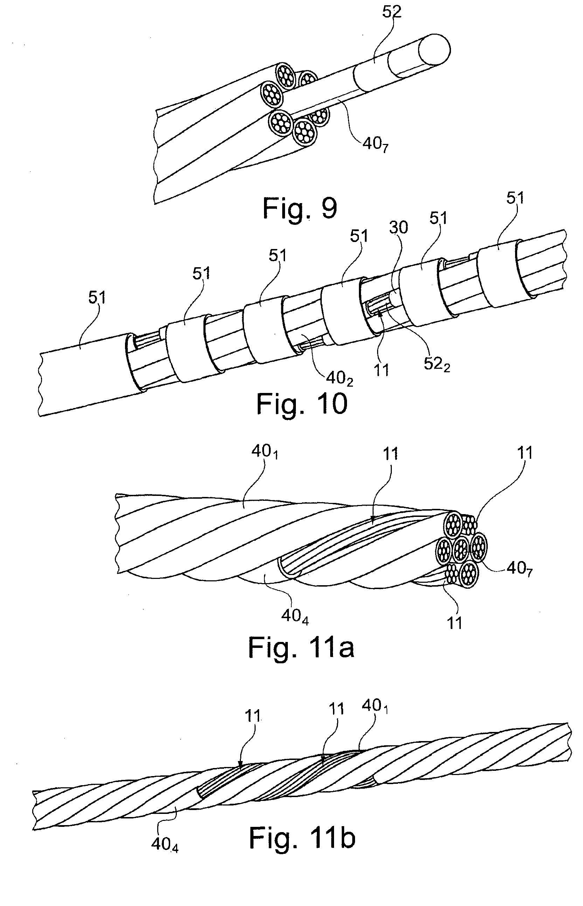

La Figure 9 montre une électrode distale 52 disposée en extrémité du microcâble central 40₇ et réalisée selon une des variantes de réalisation précédentes, notamment celle impliquant une bague intérieure et une bague extérieure.

La Figure 10 représente une version de la microsonde multipolaire dans laquelle les électrodes sont directement constituées par éléments de câbles 11 de coeur. Par exemple, l'électrode 52₂ sur la Figure 10 est la partie de câble 11 de coeur découverte à travers la zone dénudée 30 du microcâble 40₂. Dans ce cas, chaque brin élémentaire étant conducteur, son matériau doit être impérativement biocompatible, par exemple âme 1 de MP35 et enveloppe 2 en platine.

La tenue de la structure est assurée par une alternance d'éléments de gaine 51 enveloppant le toron des sept microcâbles. De préférence, la longueur dans cette alternance de la gaine extérieure est supérieure aux longueurs des intervalles. Cette gaine joue également un rôle important dans la manipulation de la microsonde, lors de l'insertion dans le cathéter. Elle assure également la mise en forme de la partie distale afin de réduire le risque de déplacement de la microsonde dans les veines.

Il est à noter que, dans cette configuration, chaque point de stimulation est orienté angulairement et ne permet pas une stimulation annulaire.

La surface d'une électrode est d'environ 0,314 mm² pour une longueur de 1 mm. Cette faible surface de stimulation est favorable à la longévité de la batterie.

Les Figures 11a et 11b représentent un dipôle basse consommation, constitué de microcâbles spiralés et recouverts chacun, dans ce cas, d'une gaine PET thermorétractée. Pour les deux microcâbles 40₁ et 40₄, on réalise au préalable une zone dénudée 30 d'une longueur correspondant à une spire (360°), ce qui permet d'augmenter la surface conductrice de chaque câble 11 de coeur à une valeur équivalant à une électrode de type bague annulaire.

La surface conductrice ainsi torsadée offre un meilleur contact avec les tissus de la veine sur 360°.

Cette architecture permet de réaliser une microsonde qui pourra être utilisée comme dipôle et obtenir ainsi une distance très faible entre les pôles. Dans cet exemple la distance entre pôles est de 0,15 mm, séparée par un microcâble central 40₇ d'épaisseur constante.

La position des deux microcâbles dénudés en périphérie peut être différente notamment par alternance de microcâbles non consécutifs tels que microcâble gainé/microcâble dénudé.

L'avantage d'un tel dispositif est de créer un champ électrique entre deux électrodes à très faible distance, de surface identique, de même matière, et se trouvant dans un même électrolyte, ce qui a pour conséquence d'augmenter l'intensité du champ électrique.

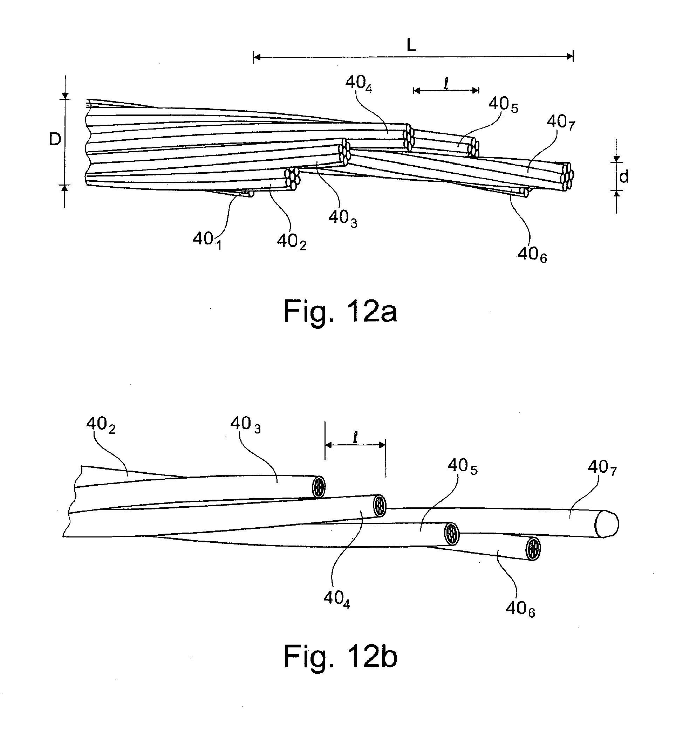

Les Figures 12a et 12b illustrent un perfectionnement des modes de réalisation décrits ci-dessus, consistant à décaler chaque extrémité de toron (et donc de microcâble) par exemple de ℓ = 1 à 5 mm de l'extrémité du toron précédent. Le résultat est une diminution progressive du diamètre de l'extrémité de la microsonde, qui dans cet exemple passe progressivement de D = 0,45 à d = 0,15 mm, sur une longueur de L = 20 à 30 mm.

Ce décalage longitudinal des extrémités des différents torons offre un double avantage :
- en premier lieu, il introduit dans la partie la plus distale de la microsonde un gradient de raideur évitant un changement brutal de diamètre entre le faisceau regroupant tous les microcâbles 40₁ à 40₇ et le seul microcâble central 40₇, ce qui réduit fortement les risques de rupture prématurée des composants sous l'effet de contraintes mécaniques de flexion ;
- par ailleurs, il améliore l'isolement électrique entre les torons, et donc l'isolement des différents pôles de la microsonde, en évitant toute proximité immédiate d'extrémités non isolées des coeurs conducteurs des différents microcâbles.

## Revendications

1. Une sonde multipolaire (50) de détection/stimulation destinée à être implantée dans des réseaux veineux, artériels ou lymphatiques,
cette sonde comprenant une pluralité de microcâbles (40₁, 40₂, 40₃, 40₄, 40₅, 40₆, 40₇),
chaque microcâble comprenant un câble de coeur (11) électriquement conducteur, apte à être relié à un pôle d'un générateur multipolaire d'un dispositif médical implantable actif, et une couche d'isolement (20) en polymère entourant le câble de coeur et comprenant au moins une zone dénudée (30) ménagée dans la couche d'isolement et destinée à former une électrode (52) de détection/stimulation monopolaire,
chaque câble de coeur étant formé par une pluralité de brins élémentaires assemblés ensemble en un toron de brins respectif,
sonde ***caractérisée en ce que** :*
- les torons de brins des différents microcâbles sont eux-mêmes assemblés ensemble en un toron de microcâbles,
ce toron de microcâbles formant une partie active distale de détection/stimulation multipolaire de la sonde, et
- la sonde est une microsonde, le diamètre de ladite partie active distale étant au plus égal à 1,5 French (0,5 mm).

2. La sonde de la revendication 1, dans laquelle les extrémités distales des microcâbles (40₁, 40₂, 40₃, 40₄, 40₅, 40₆, 40₇) sont décalées longitudinalement (ℓ) les unes par rapport aux autres,
de manière à produire une diminution progressive du diamètre et introduire un gradient de raideur dans la partie la plus distale de la microsonde.

3. La sonde de la revendication 1, comprenant un microcâble central (40₇) muni d'une électrode distale.

4. La sonde de la revendication 1, dans laquelle le câble de coeur est formé par une pluralité de brins élémentaires (10) de 0,033 mm de diamètre.

5. La sonde de la revendication 4, dans laquelle un câble de coeur de 0,1 mm est formé par un toron de sept brins élémentaires de 0,033 mm de diamètre.

6. La sonde de la revendication 4, dans laquelle les brins élémentaires présentent une structure composite constituée d'un matériau structurant (1) et d'un matériau radio-opaque (2).

7. La sonde de la revendication 6, dans laquelle le matériau structurant est un acier inox, un alliage de cobalt MP35N, un métal précieux, le titane ou un alliage NiTi.

8. La sonde de la revendication 6, dans laquelle le matériau radio-opaque est le tantale (Ta), le tungstène (W), l'iridium (Ir), le platine (Pt) ou l'or (Au).

9. La sonde de la revendication 1, dans laquelle les brins élémentaires comprennent une couche extérieure en matériau à faible susceptibilité magnétique, inférieure à 2000.10⁻¹².m³.mole⁻¹.

10. La sonde de la revendication 9, dans laquelle le matériau à faible susceptibilité magnétique est le tantale (Ta), le titane (Ti), le rhodium (Rh), le molybdène (Mo), le tungstène (W), le palladium (Pd) ou l'or (Au).

11. La sonde de la revendication 1, dans laquelle la couche d'isolement a une épaisseur de 25 µm.

12. La sonde de la revendication 1, dans laquelle le polymère constituant la couche d'isolement est un polymère fluoré.

13. La sonde de la revendication 1, comprenant une gaine (51) en polymère thermorétractable entourant partiellement la microsonde (50).

14. La sonde de la revendication 1, dans laquelle une électrode de détection/stimulation comprend une bague conductrice (52) fixée sur une zone dénudée (30) de microcâble par réduction de son diamètre extérieur.

15. La sonde de la revendication 1, dans laquelle une électrode de détection/stimulation comprend, d'une part, une bague conductrice intérieure (53) fixée sur une zone dénudée (30) par réduction de son diamètre extérieur, et, d'autre part, une bague conductrice extérieure (52) fixée sur la bague intérieure.

16. La sonde de la revendication 15, dans laquelle la bague extérieure (52) est fixée sur la bague intérieure (53) par soudure laser ou par collage au moyen d'une colle conductrice.

17. La sonde de la revendication 1, dans laquelle une électrode de détection/stimulation comprend une bague conductrice (52) fixée sur une zone dénudée (30) par collage au moyen d'une colle conductrice.

18. La sonde des revendications 14 ou 15, dans laquelle les bagues conductrices (52, 53) sont en platine iridié.

19. La sonde de la revendication 11, dans laquelle la zone dénudée s'étend sur 360°, sur une longueur correspondant à une spire de toron de microcâbles.

20. La sonde de la revendication 19, comprenant au moins un dipôle constitué de deux zones dénudées sur 360 disposées en regard sur deux microcâbles (40₁, 40₄) non consécutifs.

## Patentansprüche

1. Multipolare Sonde (50) zur Erfassung/Stimulation, die dazu bestimmt ist, in Venen-, Arterien- oder Lymphbahnen implantiert zu werden,
wobei diese Sonde eine Vielzahl von Mikrokabeln (40₁, 40₂, 40₃, 40₄, 40₅, 40₆, 40₇) umfasst,
wobei jedes Mikrokabel ein elektrisch leitendes Kernkabel (11), das geeignet ist, mit einem Pol eines multipolaren Generators einer aktiven implantierbaren medizinischen Einrichtung verbunden zu werden, und eine Isolierschicht (20) aus Polymer umfasst, die das Kernkabel umgibt und mindestens eine abisolierte Zone (30) umfasst, die in der Isolierschicht ausgenommen und dazu bestimmt ist, eine monopolare Elektrode (52) zur Erfassung/Stimulation zu bilden,
wobei jedes Kernkabel von einer Vielzahl von elementaren Strängen gebildet ist, die zu einem jeweiligen Strangbündel zusammengefasst sind,
wobei die Sonde **dadurch gekennzeichnet ist, dass**:
- die Strangbündel der verschiedenen Mikrokabel selbst zu einem Bündel von Mikrokabeln zusammengefasst sind,
wobei dieses Bündel von Mikrokabeln einen aktiven distalen Teil zur multipolaren Erfassung/Stimulation der Sonde bildet, und
- die Sonde eine Mikrosonde ist, wobei der Durchmesser des aktiven distalen Teils höchstens gleich 1,5 French (0,5 mm) ist.

2. Sonde nach Anspruch 1, bei der die distalen Enden der Mikrokabel (40₁, 40₂, 40₃, 40₄, 40₅, 40₆, 40₇) längs (1) zueinander angeordnet sind,
so dass eine progressive Verringerung des Durchmessers hervorgerufen und ein Steifheitsgradient in dem am weitesten distal befindlichen Teil der Mikrosonde eingeführt wird.

3. Sonde nach Anspruch 1, umfassend ein zentrales Mikrokabel (40₇), das mit einer distalen Elektrode versehen ist.

4. Sonde nach Anspruch 1, bei der das Kernkabel von einer Vielzahl von elementaren Strängen (10) mit einem Durchmesser von 0,033 mm gebildet ist.

5. Sonde nach Anspruch 4, bei der ein Kernkabel von 0,1 mm durch ein Bündel von sieben elementaren Strängen mit einem Durchmesser von 0,033 mm gebildet ist.

6. Sonde nach Anspruch 4, bei der die elementaren Stränge eine Verbundstruktur aufweisen, die aus einem Strukturmaterial (1) und einem röntgenopaken Material (2) zusammengesetzt ist.

7. Sonde nach Anspruch 6, bei der das Strukturmaterial rostfreier Stahl, eine Kobaltlegierung MP35N, ein Edelmetall, Titan oder eine Legierung NiTi ist.

8. Sonde nach Anspruch 6, bei der das röntgenopake Material Tantal (Ta), Wolfram (W), Iridium (Ir), Platin (Pt) oder Gold (Au) ist.

9. Sonde nach Anspruch 1, bei der die elementaren Stränge eine Außenschicht aus einem Material mit geringer magnetischer Erregbarkeit von unter 2000.10⁻¹².m³.Mol⁻¹ umfassen.

10. Sonde nach Anspruch 9, bei der das Material mit geringer magnetischer Erregbarkeit Tantal (Ta), Titan (Ti), Rhodium (Rh), Molybdän (Mo), Wolfram (W), Palladium (Pd) oder Gold (Au) ist.

11. Sonde nach Anspruch 1, bei der die Isolierschicht eine Dicke von 25 µm hat.

12. Sonde nach Anspruch 1, bei der das Polymer, aus dem die Isolierschicht ist, ein Fluorpolymer ist.

13. Sonde nach Anspruch 1, umfassend eine Hülle (51) aus wärmeschrumpfendem Polymermaterial, die die Mikrosonde (50) teilweise umgibt.

14. Sonde nach Anspruch 1, bei der eine Elektrode zur Erfassung/Stimulation einen leitenden Ring (52) umfasst, der auf einer abisolierten Zone (30) eines Mikrokabels durch Verringerung seines Außendurchmessers befestigt ist.

15. Sonde nach Anspruch 1, bei der eine Elektrode zur Erfassung/Stimulation einerseits einen inneren leitenden Ring (53), der auf einer abisolierten Zone (30) durch Verringerung seines Außendurchmessers befestigt ist, und andererseits einen äußeren leitenden Ring (52), der auf dem inneren Ring befestigt ist, umfasst.

16. Sonde nach Anspruch 15, bei der der äußere Ring (52) auf dem inneren Ring (53) durch Laserschweißen oder Kleben mittels eines leitenden Klebstoffes befestigt ist.

17. Sonde nach Anspruch 1, bei der eine Elektrode zur Erfassung/Stimulation einen leitenden Ring (52) umfasst, der auf einer abisolierten Zone (30) durch Kleben mittels eines leitenden Klebstoffes befestigt ist.

18. Sonde nach den Ansprüchen 14 oder 15, bei der die leitenden Ringe (52, 53) aus mit Iridium legiertem Platin sind.

19. Sonde nach Anspruch 11, bei der sich die abisolierte Zone auf 360° auf einer Länge entsprechend einer Bündelwindung von Mikrokabeln erstreckt.

20. Sonde nach Anspruch 19, umfassend mindestens einen Dipol, der von zwei auf 360° abisolierten Zonen, die gegenüber liegend auf zwei nicht aufeinanderfolgenden Mikrokabeln (40_{1,} 40₄) angeordnet sind, gebildet ist.

## Claims

1. A multipolar detection/stimulation lead (50) intended to be implanted in venous, arterial or lymphatic systems, this lead comprising a plurality of micro-cables (40₁, 40₂, 40₃, 40₄, 40₅, 40₆, 40₇),
each micro-cable comprising an electrically conductive core cable (11), adapted to be connected to a pole of a multipolar generator of an active implantable medical device, and a polymeric insulation layer (20) surrounding the core cable and comprising at least one naked zone (30) arranged in the insulation layer and intended to form a monopolar detection/stimulation electrode (52), each core cable being formed of a plurality of elementary conductors assembled together into a respective strand of conductors,
the lead being **characterized in that**:
- the strands of conductors of the different micro-cables are themselves assembled together into a strand of micro-cables,
this strand of micro-cables forming a distal active portion of multipolar detection/stimulation of the lead, and
- the lead is a micro-lead, the diameter of said distal active portion being at most equal to 1.5 French (0,5 mm).

2. The lead of claim 1, wherein the distal ends of the micro-cables (40₁, 40₂, 40₃, 40₄, 40₅, 40₆, 40₇) are longitudinally offset (ℓ) relative to each other,
so as to produce a progressive reduction of the diameter and to introduce a gradient of stiffness in the most distal portion of the micro-lead.

3. The lead of claim 1, comprising a central micro-cable (40₇) provided with a distal electrode.

4. The lead of claim 1, wherein the core cable is formed by a plurality of elementary conductors (10) of 0.033 mm diameter.

5. The lead of claim 4, wherein a core cable of 0.1 mm is formed by a strand of seven elementary conductors of 0.033 mm diameter.

6. The lead of claim 4, wherein the elementary conductors have a composite structure consisted of a structuring material (1) and a radio-opaque material (2).

7. The lead of claim 6, wherein the structuring material is a stainless steel, a MP35N cobalt alloy, a precious metal, titanium or a NiTi alloy.

8. The lead of claim 6, wherein the radio-opaque material is a tantalum (Ta), tungsten (W), iridium (Ir), platinum (Pt) or gold (Au).

9. The lead of claim 1, wherein the elementary conductors comprise an outer layer made of a material of low magnetic susceptibility, lower than 2000.10⁻¹².m³.mole⁻¹.

10. The lead of claim 9, wherein the low magnetic susceptibility material is tantalum (Ta), titanium (Ti), rhodium (Rh), molybdenum (Mo), tungsten (W), palladium (Pd) or gold (Au).

11. The lead of claim 1, wherein the insulation layer has a thickness of 25 µm.

12. The lead of claim 1, wherein the polymer constituting the insulation layer is a fluorinated polymer.

13. The lead of claim 1, comprising a sheath (51) made of a heat-shrinkable polymer surrounding partially the micro-lead (50).

14. The lead of claim 1, wherein a detection/stimulation electrode comprises a conductive ring (52) fixed on a naked zone (30) of micro-cable by reduction of its outer diameter.

15. The lead of claim 1, wherein a detection/stimulation electrode comprises, on the one hand, an inner conductive ring (53) fixed on a naked zone (30) by reduction of its outer diameter, and on the other hand, an outer conductive ring (52) fixed on the inner ring.

16. The lead of claim 15, wherein the outer ring (52) is fixed on the inner ring (53) by laser welding or by bonding using a conductive adhesive.

17. The lead of claim 1, wherein a detection/stimulation electrode comprises a conductive ring (52) fixed on a naked zone (30) by bonding using a conductive adhesive.

18. The lead of claim 14 or 15, wherein the conductive rings (52, 53) are made of platinum iridium.

19. The lead of claim 11, wherein the naked zone extends over 360°, over a length corresponding to one turn of a strand of micro-cables.

20. The lead of claim 19, comprising at least one dipole consisted of two zones naked over 360°, arranged opposite to each other on two non-consecutive micro-cables (40₁, 40₄) .
